Europäisches Patentamt

European Patent Office

Office européen des brevets

(11) Publication number: **0 052 208**
A2

# EUROPEAN PATENT APPLICATION

(21) Application number: 81107857.5

(22) Date of filing: 02.10.81

(51) Int. Cl.³: **C 11 B 1/06**
**C 12 R 1/07**

(30) Priority: 02.10.80 PH 24657

(43) Date of publication of application:
26.05.82 Bulletin 82/21

(84) Designated Contracting States:
AT BE CH DE FR GB IT LI LU NL SE

(71) Applicant: Ramos, Milagros Atacador
11 Dukes Street Greenhills
San Juan Metro Manilla(PH)

(71) Applicant: Puyat, Gil G., jr.
1564 Mahogany Street Dasmarinas Village
Makati Metro Manilla(PH)

(71) Applicant: Tantoco, Hermogenes L.
1564 Mahogany Street Dasmarinas Village
Makati Metro Manilla(PH)

(72) Inventor: Ramos, Milagros Atacador
11 Dukes Street Greenhills
San Juan Metro Manilla(PH)

(72) Inventor: Puyat, Gil G., jr.
1564 Mahogany Street Dasmarinas Village
Makati Metro Manilla(PH)

(72) Inventor: Tantoco, Hermogenes L.
1564 Mahogany Street Dasmarinas Village
Makati Metro Manilla(PH)

(74) Representative: Patentanwälte Beetz sen. - Beetz jr.
Timpe - Siegfried - Schmitt-Fumian
Steinsdorfstrasse 10
D-8000 München 22(DE)

(54) Process for the production of coconut oil.

(57) The invention relates to a process for the production of coconut oil from fresh coconuts comprising the following steps:
dehusking the coconut, splitting the coconut, collecting the coconut water, separating the coconut meat, grinding the meat, separating the meat and a first milk extract, adding coconut water to the pressed meat and mashing and pressing this mixture to separate the meat and the milk extract as well as repeating this extraction step twice to produce second and third milk extracts, separating the cream from the skimmed milk by setting aside said extracts for one or two hours, adding skimmed milk as a diluent to said cream and adjusting the pH-value to 6.0 - 6.5, adding a cultured microorganism and fermenting the mixture and separating the white oil and the curd. These products provide an excellent and cheap source of energy.

Process for the production of coconut oil


This invention relates in general to the production of coconut oil. More specifically, it relates to a process for the production of coconut oil from fresh coconutswherein the extraction is done under controlled conditions by the use of microorganism.

Coconut, the Philippines' major commercial crop, is considered as the cheapest source of energy and plant protein. It is processed into a variety of products - mainly for food and industrial uses. Coconut products which are currently under extensive research include coconut oil, coconut protein isolate, coconut skim milk and coconut flour.

Extraction of oil from coconut meat can be classified under the "DRY" and the "WET" processes. For better enlightenment on the techniques of oil extraction, as gathered from the previous investigations, the different ways shall be described briefly hereunder:

The main steps involved in the processing of coconut under the "DRY" process are:

0154-(24657)-Ms

1. Comminution of fresh coconut meat;

2. Drying of coconut meat; and

3. Extraction of oil by the use of expellers.

Several investigators have patented their findings on this type of processing coconut oil, among them are Hiller, Yenko, Carver-Greenfield, and the NIST Integrated Coconut Processes. Actually, the basic principle is the same but differ only in the details involved. Oil is extracted after the meat is comminuted and dried, whereas in the WET process the oil is extracted from the aqueous mixture of the water soluble constituents of the coconut meat.

1. Hiller process - In this process, the mature nut is cooked, then the kernel is removed from the shell and sliced. The sliced kernel is rapidly dried and the oil extracted with the use of the expeller. The cake is ground into a meal.

2. Modified Miller process - The Rogers engineering group has tried the modification of the Miller process using a more sophisticated method of drying the comminuted coconut meat, using a batch drier under vacuum the purpose being to subject the meat to a less sever heat treatment. This method, has been done only on an experimental basis and has not gone into pilot nor commercial scale production.

3. Yenko process - The processes involved are selection of the nut, splitting into halves, removal of the shell, comminution of the meat, heating of the meat at 80 to 100 $^{\circ}$C, pressing - resulting in the release of oil which is then filtered and the cake with 1 to 8% moisture is grounded into coconut flour.

4. Carver-Creenfield process - In this method, the important feature is the evaporation of the water in the ground meal at low temperature in the presence of excess of oil followed by pressing the slurry to separate the oil from the non-fat constituents.

5. NIST Integrated Coconut Process - The process consists of grinding the meat in a colloid mill and drying into a fluidified bed drier. The resulting protein-rich product is granulated coconut.

6. Drying and Expelling - The process involved drying and expelling process resulting in flour with comparatively high fat content (12% - 15%).

The WET process of Lava, Birosel, Robledano-Luzuriaga, Cruz-Bernardo, Gonzaga Diokno, Central Food Technological Research Institute (CFTRI) Azeotropic, Krauss-Maffei, Chayen, and Hagenmaier differ only in the details of the principal unit operation which are:

1. Comminution of fresh coconut meat;

2. Extraction of oil from the aqueous mixture of the water soluble constituents of the coconut meat; and

3. Separation of oil from the liquid extract as recovery of the resulting product.

In general there are some objections to the wet process, such as yield of the oil is very low, the water soluble protein passes out with the liquid extract from fresh coconut meat and there are still mechanical defects in the process. As of now none of the wet processes is in commercial application although researches

- 4 -

are still going in order to improve the oil yield and to produce a protein-rich meal suitable for human consumption and to make the processing economically feasible. The <u>WET</u> process is described briefly as follows:

1. <u>Gonzaga process</u> - The coconut meat is grated and pressed, then the liquid is placed in a suitable container (preferably a tank) to settle. The cream is heated at 100 $^{\circ}$C to produce the oil and the coagulated solids. This method was patented in 1948.

2. <u>Lava process</u> - The oil is extracted from fresh grated coconut meat by water extraction and subsequent separation of oil from the protein. The by-products are the pressed cake and an aqueous sugar-mineral solution. The dried protein concentrate could be used as human and/or animal feeds. This process was developed in 1937 and patented in the U.S. in the same year; in France in 1938 and in Britain in 1939.

3. <u>Diokno process</u> - This process consists of separation of the oil and the solids by a series of centrifugation steps. The solids are dried and ground, and the end product is a high protein solid product. The process was patented in 1960.

4. <u>Robledana - Luzuriaga process</u> - The fresh coconut meat is ground and pressed to yield a solid residue and the emulsion is subjected to control enzymatic action, frozen and thawed to separate the oil by subsequent centrifugation. Heating the water phase yields the protein concentrate. The residual meal is reported to be suitable for direct human consumption. The process was patented in 1948.

5. Central Food Technological Research Institute (CFTRI)Azeotropic Extraction - The process consists of separating the kernels from the shells, milling and mixing with heptane solvent. The extraction is carried out until the material is free from moisture and oil. The meal is then passed through a micro-atomizer and powdered. It can be used as such in edible formulations. The process was developed by the Indian Central Food Technological Institute of Mysore.

6. Chayen process - This process consists of feeding fresh coconut kernels into the impulse renderer with ten times their weight of a 0.15% sodium hydroxide solution. The carbohydrate fiber fraction is removed on a vibrating screen and the liquor passing forward is centrifuged to separate the free oil from the lipid-protein complex. The oil and the complex are recovered by methods similar to those used in peanut processing. The free oil which accounts for more than three quarters of the total lipid of the kernels, after one washing only, separates an almost colorless solid of low free fatty acid content and with a faint odor of coconuts. About three quarters of the nitrogen of the original kernel is recovered as protein in the lipid-protein complex. The dried complex is almost odorless and tasteless. It is a free flowing brown powder of pleasing appearance.

7. Kraus-Maffei (KM) and CFTRI/KM - These processes are similar in principle to the Robledano process. The kernel obtained from steamed coconut is separated from the shell, passed through a cutter and rollers, and passed through a screw press. The milk freed of fibrous meal is separated into oil phase and water phased by centrifugation. The waterphase is concentrated to produce "coco honey" which is rich in protein that can be coagulated by heat or acid. In the modified CFTRI/KM process the streaming of the nut is omitted and the recoveries of oil and protein are improved.

8. Tropical Product Institute (TPI) process - The TPI process is based on an initial disintegration of coconut meats into a slurry which is filtered or screened to remove the fiber, then centrifuged after adjustment of the pH to that which is isolectric for coconut protein. At this pH most of the protein is not in solution and this minimizes the problem of emulsions. Consequently, the process yields oil, protein and water-solubles in three separate product streams. The process also yielded a fourth phase which is an emulsion containing a substantial quantity of both oil and protein.

The pressing need for processing coconut into oil of high quality with subsequent production of quality proteins for human consumption led the present inventor to make further studies on the technology of oil production, considering the fact that the use of expellers is disadvantageous because it generates heat which destroys the nutritive value and adversely affects the color of meal.

The result of these investigations is the present process which is called CASAMIL PROCESS which has vastly improved the production of high quality oil and protein concentrates for human consumption. This process likewise provides a number of by-products which include the sapal (coconut meal residue) and protein solids which if not properly processed or disposed of could cause pollution and sanitation problems.

Among the various methods of extracting oil from coconut meat, the expeller type is the most common. But this process usually yields an oil which is colored and easily becomes rancid. Because of this deficiency, the oil cannot be used directly for industrial purposes and therefore as a corrective measure it has to be bleached and deodorized before it can be used in the manufacture of industrial products like hydrogenated oil, pomades, pharmaceuticals and other culinary preparations.

To improve the quality of coconut oil, several processes had been patented, most of them based on the WET or coconut milk process. The milk is extracted from fresh coconuts and subjected to various treatments which take a number of days to complete. During this period, unfavourable re-action usually takes place on the milk substance which adversely affects the quality of the oil. Free fatty acids content increase when the oil is stored for long. Thus, the oil must be refined before it becomes suitable for the manufacture of edible products. The entails substantial expenditure, which be avoided for if the CASAMIL PROCESS herein presented is adopted. The energy costs expended in the refinery of coconut oil is very exorbitant nowadays - and this could be saved because the CASAMIL way uses no heating.

Milk as extracted from coconut meat is an emulsion of coconut oil, water, sugar, protein and salts. The proteins are colloidally dispersed at the water-oil interface and the sugars, mostly dissolved in the aqueous phase, act as emulsifying agents. At this point, the most ideal would be to find a means of breaking the emulsion to liberate the oil without affecting the quality of the oil.

The CASAMIL process breaks efficiently the emulsion in the milk to produce a good amount and quality of oil.

A number of technical methods have been tried for breaking emulsions with the use of centrifugal force, electrical energy, filtration under pressure and heat treatment. All to no avail - even with the assistance of expensive equipment - because they could not attain the desired quality of the oil.

Since the sugars act as emulsifying agents, the problem would be to find a way of recovering the oil without affecting adversely its quality and purity by destroying that materials that act as emulsifiers. If a substance therefore could be found which when added to the coconut milk would break down the sugar only, emulsion might be broken and the oil liberated. Another alternative is to use microorganisms to break the emulsion to release the oil.

A type of microorganism had been isolated by the inventor and this is the de-emulsifying agent used in this oil recovery process. Known as the "CASAMIL" process this new oil extraction is simple, economical and easy to operate.

The primary object therefore of the present invention is to provide a process for the production of coconut oil from fresh coconuts which would increase the yield and improve the grade of coconut oil and at the same time produce quality proteins.

- 9 -

Another object of this invention is to provide a process for the production of coconut oil which would minimize, if not totally eliminate, the use of heating (which comprises almost 75% of the cost of production), currently practiced by processors.

Still another object of the present invention is to provide a process for the production of coconut oil wherein the extraction is done under controlled conditions by the use of microorganism.

Other objects and advantagous features inherent in this invention will become apparent from the reading of the specification which will hereinafter be described in detail.

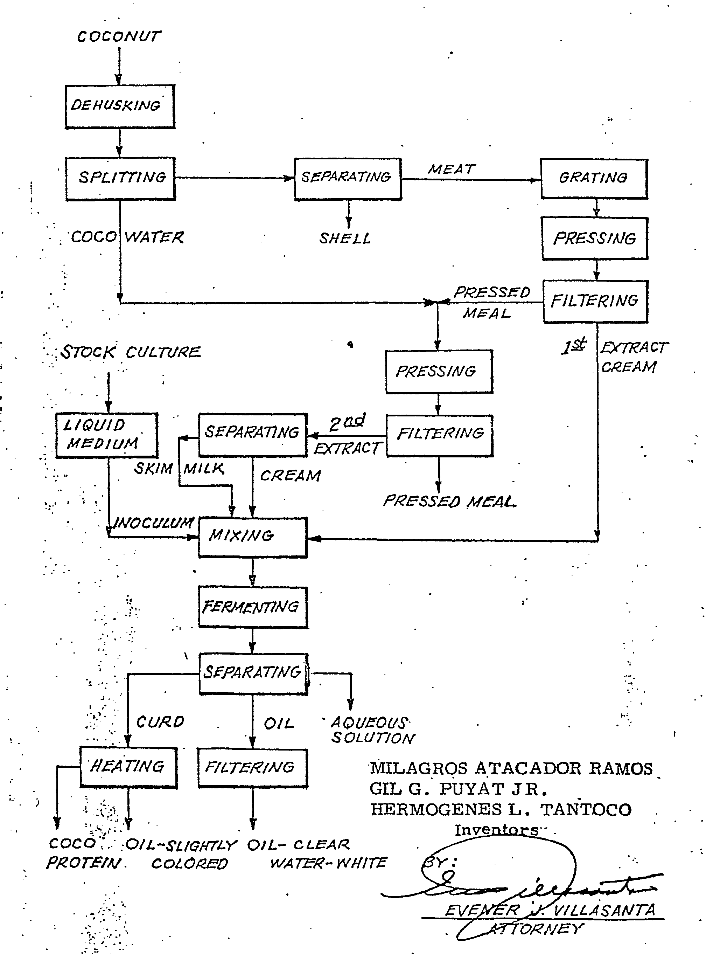

The flow diagram of the present process is shown in the accompanying drawing.

Sound matured coconuts were selected, dehusked and cut into halves and the coconut water is collected. The meat is separated from the shell and the meat is then thoroughly ground and mashed and pressed or squeezed to separate the first milk extract and the meal. To the pressed meal (sapal) an equal amount of coconut water was added and again mashed and squeezed as before. This extraction was repeated twice to get all the milk. The first, second and third extracts were set aside for an hour or two to separate the cream from the skimmed milk.

The organism used in this study is a spore-forming "bacillus spp." (CMR-70) and propaged in sterile nutrient boullion.

As inoculum or starter, a 24-48 hours old culture of "bacillus spp." (CMR-70) in a liquid medium was used.

The separated coco cream was placed in an Erlenmeyer flask into which an equal volume of skimmed milk was added as diluent and the pH adjusted to pH 6.0-6.5. A 10% of the microbial starter was used as inoculum. The inoculated milk was incubated at 30-40 $^{O}$C for 12-24 hours. Fermentation started after 6 hours and proceeded progressively. After 12-24 hours a clear separation of the oil and the rest of the water and curd occurred. The oil was harvested and the curd separated.

Based on the results of several trials it was evident that the cultured organism CMR-70 has the ability of de-emulsifying the milk capable of breaking emulsion to release the oil and attain effective recovery.

From the initial volume of coco cream used, a 50-60% superior grade water-white oil was produced. It was practically odorless and was found to be negative to the test for rancidity.

Compared with other processes, the oil obtained either by rendering or by the use of expellers was of an inferior grade. The oil was colored, the shade being dependent on the manner in the boiling had been done. It had also a pronounced nutty odor.

From a series of experiments undertaken, the following important observation was made. The grade of the oil was dependent on the quality and degree of ripeness of the nuts; on the amount of pressure used in extracting the coconut milk from the ground meat; on the state of subdivision of the meat; and on the purity and viability of the microorganism.

With the CASAMIL process using "bacillus spp." (CMR-70) the yield of superior grade of coconut oil was materially increased from 24.79% to 50-60% while yield of this grade of oil in the absence of microorganisms was very much less (0-20% only).

The curd which still contained oil was transferred into a beaker and carefully heated until the protein was completely coagulated. An additional yield of an inferior grade of oil was obtained. The oil was slightly colored and had a mild nutty odor. The intensity of the color was dependent on the temperature and on the length of time at which the curd was heated.

The yield of inferior grade of oil (obtained from heating the curd) when the organism was introduced ranged from 1.90% to 15% while in the absence of the organism the yield was from 9.20% to 40%. A 5% to 15% curd was recovered. The curd contained a fairly high amount of protein - about 72%. In view of its high protein and mineral content, it could be used as a nutritional supplement to our dient and for enriching snack foods, etc. or as a basic protein component of a multi-purpose food formulation.

Characteristics of the coconut oil

| | |
|---|---|
| Sp. gr. at 29 $^{o}$C | 0.9180 |
| Sap. value | 258 |
| Iodine value | 7.3 |
| Acid value | 5.25 |
| M. Pt | 24.5 $^{o}$C |
| Free fatty acids | 2.64 |
| Color | 133.4 w/m - 0.5 y/O.IR |
| POV | 0.6 |

### Chemical composition of the curd (coconut protein)

| Proximate | % |
|---|---|
| Moisture | 6.80 |
| Protein | 72.00 |
| Fat | 0.80 |
| Ash | 4.70 |
| Crude fiber | 0.20 |
| Carbohydrate | 15.40 |

| Mineral Elements | mg% |
|---|---|
| Calcium | 150 |
| Phosphorus | 1,095 |
| Iron | 18 |
| Magnesium | 540 |

### Amino acid composition of curd (coco protein) g/16g nitrogen

| | | |
|---|---|---|
| Arginine | 12.00 | Tyrosine |
| Histidine | 2.60 | |
| Valine | 6.30 | |
| Methionine | Traces | |
| Threonine | 4.50 | |
| Phenylalanine | 6.20 | |
| Glutamic acid | 21.00 | |
| Isoleucine | 4.04 | |
| Leucine | 7.05 | |
| Lysine | 4.06 | |
| Tyrosine | 1.00 | |
| Alanine | 5.13 | |

What we claim is:

1. A process for the production of coconut oil from fresh coconuts comprising the steps of:

   a) dehusking the coconut,

   b) splitting the coconut in halves and collecting the coconut water,

   c) separating the coconut meat from the shell,

   d) thoroughly grinding the meat and processing the same to separate the meal and the first milk extract,

   e) adding an equal amount of coconut water to the pressed meal and mashing and pressing the mixture to separate the meal and the milk extract and repeating this extraction twice, to produce the second and third milk extracts,

   f) setting aside the first, second and third extracts for one or two hours to separate the cream from the skimmed milk,

   g) adding an equal volume of skimmed milk as a diluent to the separated coconut cream and adjusting the pH to 6.0 - 6.5,

   h) adding a 10% of the cultured microorganism "bacillus spp." (CMR-70) and fermenting the mixture, and

   i) separating the clear water-white oil produced and the by-product curd.

- 2 -

2. The process according to claim 1, wherein the
   curd produced in step (i) is carefully heated
   until the protein is completely coagulated and
   the slightly colored oil produced is separated.

COCONUT → DEHUSKING → SPLITTING → SEPARATING —MEAT→ GRATING → PRESSING → FILTERING

SPLITTING → COCO WATER

SEPARATING → SHELL

FILTERING → 1st EXTRACT CREAM

PRESSED MEAL → PRESSING → FILTERING → PRESSED MEAL

2nd EXTRACT

STOCK CULTURE → LIQUID MEDIUM

SEPARATING → SKIM MILK / CREAM

INOCULUM → MIXING → FERMENTING → SEPARATING

CURD / OIL / AQUEOUS SOLUTION

HEATING → COCO OIL-SLIGHTLY PROTEIN COLORED

FILTERING → OIL-CLEAR WATER-WHITE

MILAGROS ATACADOR RAMOS
GIL G. PUYAT JR.
HERMOGENES L. TANTOCO
Inventors

BY:

EVENER J. VILLASANTA
ATTORNEY